# EUROPEAN PATENT APPLICATION

(11) **EP 0 919 231 A1**
(43) Date of publication of application: **02.06.1999**
(21) Application number: 97500161.1
(22) Date of filing: 15.10.1997
(51) Int. Cl.: A61K 31/415, A61K 47/10

(54) **Injectable parasiticide composition and process for the preparation**

(71) Applicant: Biogenesis, S.A., 1022 Buenos Aires (AR)
(72) Inventor: Lamberti, Jorge Carlos, 1430 Buenos Aires (AR)
(74) Representative: Ponti Sales, Adelaida

(57) **Abstract**

The invention relates to a novel injectable parasiticide composition, comprising a compound of formula (I) wherein R is a propyl (ricobendazole) or a phenyl (oxphendazole) group, preferably to be used in the veterinary field, in a concentration of about 10% (w/v) in relation to the final volume, a first solvent selected from ethanol and isopropanol, an inorganic acid and the required quantity of propylene glycol up to the 100% of the final volume.

The process for its preparation comprises adding to a first solvent the sufficient amount of said compound of formula (I) to achieve a final concentration of about 10% (w/v), followed by the addition of an inorganic acid; heating at a temperature ranging from 45° to 70°C until total dissolution, adding the required amount of propylene glycol up to the 100% of the final volume.

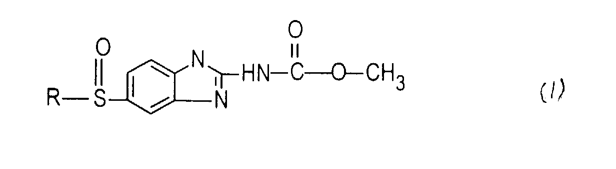

## Description

### Field of the invention

The present invention is related to an injectable antiparasitary composition and a process for its preparation. More particularly, the present invention refers to a new injectable composition of a benzimidazole derivative having the formula (I): 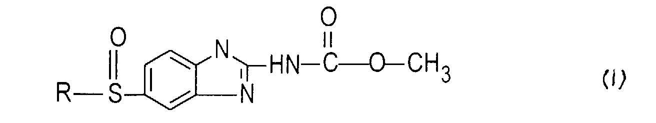 where R is a propyl or phenyl group.

### Background of the invention

The search of antiparasitary compositions has been of relevant interest for the veterinary pharmaceutical industry. The development of antihelminthic compounds having better quality for the control and treatment of the gastrointestinal and pulmonary diseases caused by the nematodes which are of the greatest impact on bovine cattle has been a relevant aim.

The first important progress in this field took place in 1938 with the phenotiazin discovery. When the thiabendazole was brought into the market in 1961, the first antiparasitary based on benzimidazole was introduced and constitutes a new and important advance of the veterinary pharmaceutical industry for the development of antihelminthic compounds with better qualities.

Desirable features of an effective antiparasitary compound are:
a) Broader spectrum of antiparasitary activity,
b) Reasonable cost,
c) Doses which do not cause side effects,
d) Practical way of administration, and
e) Brief period of restriction post-treatment.

From the sixties, when the thiabendazole was discovered, several modifications that have been made to the benzimidazole molecule allowed the appearance of different compounds that belong to the group of benzimidazoles. Various chemical changes in position 2 and 5 of the ring system of the molecule produced the strongest drugs of the group. Mainly, those that contain an atom of sulphur in position 5, such as phenbendazole and albendazole, with high effectiveness against gastrointestinal and pulmonary nematodes, both adults and inhibited larvae and eggs.

When a benzimidazole is administrated by enteral way, as a suspension form, the particles must dissolve in the gastrointestinal fluids, in order to favor the absorption of the active principle through the gastrointestinal mucus. Mainly, this can be observed in ruminants, since the ruminal bacteria metabolize the benzimidazoles, thus making them more effective. More than one dose must be applied in order to achieve the same effect in monogastric animals.

Drugs with more hidrosolubility such as thiabendazole and cambendazole, dissolve totally in the ruminal fluid and are quickly absorbed. The more modern benzimidazole compounds such as albendazole, phenbendazole and their active metabolites show poor solubility in the gastrointestinal fluids, therefore the dissolution process trough the gastrointestinal tract and the absorption to the systemic circulation are slower. That assures a longer permanence in the organism, thus determining more efficacy.

When administering benzimidazoles by oral or intraruminal way, the rumen acts as reserving, thus extending the absorption process of the administered drug which produces an increase of the bioavailability thereof. Said process can be affected because of metabolic, functional, nutritional or physiological changes (esophagus leak, bridge of fodder, ruminal flora, etc.)

The distribution in the body of the animal when treated with a benzimidazole is under the influence of the administration way, the molecular weight, the affinity in bonding with plasmatic proteins and the liposolubility.

At plasmatic pH, the benzimidazoles are very liposolubles, thus favoring their distribution. The recycle produced between plasma and digestive tract is a fundamental factor of the efficacy of the benzimidazoles. The abomasal and intestinal parasites are more exposed to benzimidazole because of the drug that recycles between the plasma and the gastrointestinal tract, than the drug not absorbed being dissolved into the gastrointestinal fluids. Henneisy, D and Prichard, R.K. proved that the oxphendazole administered by intravenous way is more effective against Haemonchus contortus and Trichostrongylus colubriformis, which are resistant to benzimidazoles, than those orally administrated.

Albendazole, Phenbendazole and their sulfoxides Ricobendazole and Oxphendazole, are very lipophilic at plasmatic pH, giving them an extended permanence in the systemic circulation. It means an improvement in the availability for the recycle between the plasma and the gastrointestinal tract, determining that the parasites at gastrointestinal location are subjected to therapeutical levels of drug for a longer time.

The benzimidazoles undergo metabolic processes in several organs. Said processes are of reversible nature (sulfoxidation), giving rise to metabolites with antiparasitary effect and others of irreversible nature (sulfonation), giving rise to inactive metabolites.

The metabolites from albendazoles and phenbendazoles are interchanged in a reversible way between plasma and gastrointestinal compartments through a distribution process which is dependant on a pH gradient.

When administering albendazole or phenbendazole to bovines, the metabolites ricobendazole or oxphendazole respectively are detected in plasma, so far as 36 hours postreatment, while the albendazole and the phenbendazole themselves are not detected in the plasma, but in the lumen gastrointestinal. Therefore, Ricobendazole and Oxphendazole are the active metabolites from albendazole and phenbendazole respectively, against pulmonary vermes, inhibited larvae and immature fasciolae, being preferred due to their higher antiparasitary efficacy.

Ricobendazole and Oxphendazole show high efficacy in the control of gastrointestinal and pulmonary parasitism that affect bovine cattle. Particularly against
Haemonchus sp., Ostertagias sp., Trichotrongylus sp., Cooperaia sp., Dictyocaulus sp..

As above expressed, when the benzimidazoles are administered by enteral way, they are metabolised in the rumen, being later absorbed by the gastrointestinal mucus, passing to the plasma where they carry out the antiparasitary action. This process can be influenced by several factors such as physiological, functional or metabolic that can produce changes to the drug into the pre-stomachs of the ruminants. Among the physiological factors to be taken into account, the closing of the esophagus leak, mainly in young animals, causes the benzimidazoles to introduce directly into the abomasum, wherein most of the drug is inactivated. Another physiological factor to bear in mind is the bridge of fodder in the rumen, where the benzimidazoles could be sequestrated, when the diet is very fibrous, thus not rendering the desired plasmatic concentration level in order to carry out the antiparasitary action. Therefore, in order to achieve effectiveness of the antiparasitary action, the cattle is subjected to previous fast, with the consequent loss of weight.

The loss of drug by means of both ebb and changes of the gastrointestinal microflora are other drawbacks of the orally dosage of the product.

### Description of the invention

Therefore, the main object of the present invention is to provide an injectable parasiticide composition of a compound of formula (I): 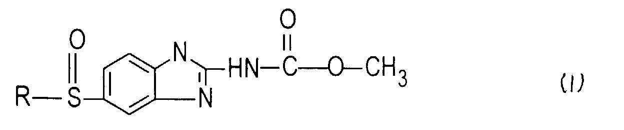 wherein R is a propyl or phenyl group. More specifically, the methyl ester of [(5-propyl-sulphinyl)- 1H-benzimidazol-2-il] carbamic acid (Ricobendazole) and the methyl ester of [(5-phenyl-sulphinyl)- 1H-benzimidazol-2-il] carbamic acid (Oxphendazole).

Advantageously, an injectable composition, subcutaneous injectable composition, for example, besides the simplicity of its application also provides important benefits with respect to the oral compositions. In fact, the above mentioned physiological, functional and metabolic factors (oesophagus leak, bridge of fodder, ruminal flora, etc.) that can alter the drug in the pre-stomachs of the ruminants are avoided. On the other hand, product loss because of ebb, as usually happens with oral dosage, are avoided.

Likewise, as above mentioned, the subcutaneous administration of oxphendazole or ricobendazole allows their fast absorption by plasma, reaching high concentrations, thus obtaining optimum therapeutical levels in the sites where the parasites are located, developing a direct action against pulmonary vermes, inhibited larvae, immature fasciolae, and eggs.

During the research in order to obtain a suitable formulation, clinical tests in animals were made, as is shown below. The Applicant has determined that unexpectedly the optimum concentration of the compound having the formula (I) in an equilibrated formulation must be of about 10% (w/v). It was observed that a higher concentration value, such of around 15% produced edemas and abnormal accumulation of serous fluid in subcutaneous tissue, in the animals subjected to study.

On the other hand, not less important was the exclusion of butylic alcohol from the present new composition. Clinical assays in sheep have shown that the presence of butanol in injectable compositions produced edemas and accumulation of serous fluid in subcutaneous tissue at the inoculation point.

Therefore, the object of the present invention is an injectable parasiticide composition of a compound of formula (I) that comprises said compound of formula (I) which is present in a concentration of about 10% (w/v) with respect to the final volume, a first solvent selected between ethanol and isopropanol, an inorganic acid and the required quantity of propylene glycol to reach 100% of final volume.

Preferably, the ricobendazole or oxphendazole compound is incorporated in the composition in enough quantity to achieve a final concentration ranging from values of 9 to 11% (w/v).

More preferably, the ricobendazole or oxphendazole compound is incorporated to the composition in enough quantity to achieve a final concentration of 10% (w/v).

The following examples are given for the purpose of illustrating the present invention and for its better understanding and are not to be considered as limiting the invention set forth in the claims.

### EXAMPLES

### Example 1 - Preparation of a ricobendazole composition

10 Kg of methyl ester of [5-propyl-sulphinyl-1H-benzimidazol-2-il] carbamic acid (ricobendazole) (100%) and 3 liters of hydrochloric acid are added to 20 liters of ethanol. The mixture is heated at a temperature ranging from 45-70°C, with agitation until the ricobendazole is totally dissolved. The volume is increased up to 100 liters with propylene glycol.

### Example 2 - Preparation of an oxphendazole composition

10 Kg of methyl ester of [5-phenyl-sulphinyl- 1H-benzimidazol-2-il] carbamic acid (oxphendazole) (100%) and 3,5 liters of hydrochloric acid are added to 20 liters of ethanol. The mixture is heated at a temperature ranging from 45-70°C, with agitation until the oxphendazole is totally dissolved. The volume is increased up to 100 liters with propylene glycol.

### Example 3 - Clinical Assays

A group of 46 steers A. Angus and Hereford were split at random in two groups.
Product Lot: 40 animals
Control Lot: 6 animals

The average weight of the steers was 250 kg. During the first 30 days of the assay the availability of forage was good (perennial pasture, such as agropyron, festuca and alfalfa). During the last 30 days because of lack of rain and high temperatures, the forage quality decreased (dry agropyron).

Once the lots were arranged, the animals were weighted and identified. Further, a sample of faecal matter was taken from each animal for its coproparasitologycal analysis expressed as EPG values (eggs of parasites per gram of faecal matter).

Continuously and at the same day of the taking of samples, the animals of the Product Lot were inoculated subcutaneously with a dose of the composition of ricobendazole as obtained in Example 1. The dose was of 5 ml/100 Kg. of alive-weight. No more than 10 ml were injected in one point. The Control Lot of 6 animals does not receive any treatment.

On the 5th day post-treatment several animals were taken at random from the Product Group and all the animals from the Control Group. New samples of faeces were taken, cooling them for their analysis. Also the injected areas were examined; no nodules were observed.

30 days after the first treatment, the animals were weighteds and a sample of faecal matter was taken. A second dose of the antiparasitary composition was administered, like in the first treatment.

On the 5th day after the second treatment new faecal matter samples were taken for their analysis. Also the injected areas were examined; no nodules were observed.

60 days after of the first treatment, the weight of the animals was checked.

The resulting values are shown in the following tables: Product Group Table, Control Group Table, Results Table,
where:
EPG1 : Day 0 (first dose)
EPG2: Day 5 (analysis after the first treatment)
EPG3: Day 30 (second dose)
EPG4: Day 35 (analysis after the second treatment)

| PRODUCT GROUP Table | | | | | | | |
|---|---|---|---|---|---|---|---|
| ANIMAL | WEIGHT | EPG1 | EPG2 | WEIGHT | EPG3 | EPG4 | WEIGHT |
| 1 | 232 | | | 255 | | | 284 |
| 2 | 228 | | | 251 | | | 263 |
| 3 | 244 | | | 268 | | | 284 |
| 4 | 236 | | | 255 | | | 277 |
| 5 | 347 | 440 | 0 | 362 | 80 | 0 | 388 |
| 6 | 212 | 220 | 60 | - | 40 | 0 | 262 |
| 7 | 250 | 360 | 0 | 295 | 60 | 0 | 315 |
| 8 | 283 | | | - | | | - |
| 9 | 238 | 260 | 20 | 285 | 80 | 0 | 293 |
| 10 | 287 | | | 345 | | | 385 |
| 11 | 216 | 50 | 0 | 237 | 40 | 0 | 262 |
| 12 | 257 | | | 300 | | | 318 |
| 13 | 241 | | | 275 | | | - |
| 14 | 248 | | | 277 | | | - |
| 15 | 208 | | | 230 | | | 250 |
| 16 | 235 | | | 288 | | | 313 |
| 17 | 262 | | | 275 | | | 315 |
| 18 | 287 | 480 | 60 | 305 | 0 | 0 | 326 |
| 19 | 270 | | | 260 | | | 315 |
| 20 | 240 | | | 278 | | | 305 |
| 21 | 216 | 40 | 20 | 265 | 20 | 0 | 281 |
| 22 | 223 | | | 248 | | | 277 |
| 23 | 250 | | | 273 | | | 300 |
| 24 | 255 | 20 | 0 | 302 | 0 | 0 | 330 |
| 25 | 248 | 40 | 40 | 307 | 0 | 0 | 340 |
| 26 | 240 | 180 | 20 | 285 | 20 | 0 | 302 |
| 27 | 262 | 40 | 0 | 297 | 0 | 0 | 319 |
| 28 | 279 | 200 | 0 | 303 | 0 | 0 | 319 |
| 29 | 220 | 680 | 40 | 248 | 20 | 0 | 283 |
| 30 | 261 | | 0 | 294 | 60 | 0 | 315 |
| 31 | 190 | | | 224 | | | 255 |
| 32 | 227 | | 20 | 260 | 40 | 0 | 300 |
| 33 | 245 | | | 267 | | | 295 |
| 34 | 234 | | | 283 | | | 289 |
| 35 | 228 | | 0 | 247 | 0 | 0 | 280 |
| 36 | 267 | | | 305 | | | 319 |
| 37 | 245 | | | 272 | | | 300 |
| 38 | 255 | 60 | 20 | 297 | 20 | 0 | 325 |
| 39 | 279 | 500 | 40 | 299 | 40 | 0 | 345 |
| 40 | 224 | | | 285 | | | 304 |

| CONTROL GROUP Table | | | | | | | |
|---|---|---|---|---|---|---|---|
| ANIMAL | WEIGHT | EPG1 | EPG2 | WEIGHT | EPG3 | EPG4 | WEIGHT |
| 91 | 237 | 160 | 200 | 275 | 100 | 80 | 302 |
| 92 | 250 | 280 | 160 | 261 | 120 | 60 | 290 |
| 93 | 253 | 180 | 220 | 273 | 160 | 40 | 305 |
| 94 | 272 | | | 287 | | | 312 |
| 95 | 288 | 100 | 60 | 301 | 60 | 20 | 327 |
| 96 | 263 | 120 | 100 | 291 | 40 | 60 | 310 |

| RESULTS Table | | | | |
|---|---|---|---|---|
| | PRODUCT GROUP | DIFFERENCE | CONTROL GROUP | DIFFERENCE |
| INITIAL | | | | |
| WEIGHT | 9846 Kg | 0 | 1536 Kg | 0 |
| | 246 Kg | | 261 Kg | |
| | | | | |
| Total | | | | |
| | | | | |
| Average | | | | |
| 30 DAYS | | 32.80 Kg | | 20,83 Kg |
| WEIGHT | 10600 Kg | | 1688 Kg | |
| | 278.95 Kg | | 281.33 Kg | |
| | | | | |
| Total | | | | |
| | | | | |
| Average | | | | |
| 60 DAYS | | | | |
| WEIGHT | 11239 Kg | 24.81 Kg | 1846 Kg | 26.33 Kg |
| | 303.76 Kg | | 308 Kg | |
| | | | | |
| Total | | | | |
| | | | | |
| Average | | | | |
| TOTALS | | 57.60 Kg | | 47.16 Kg |
| Differences treated/control | | 10.50 Kg | | 0 |

### Example 4 - Study of cutaneous irritability in rabbit

The appearance of irritation and/or edema after the subcutaneous injection of the ricobendazole composition obtained according to Example 1 with an active drug concentration of 10% (w/v) was determined and quantified.

A group of 6 rabbits was inoculated with a subcutaneous dose of said composition at a ratio of 0,05 ml/Kg of rabbit.

No edema was observed in any rabbit.

### Example 5 - Study of cutaneous irritability in rats

The appearance of irritation and/or edema after the subcutaneous injection of the oxphendazole composition obtained according to Example 2 with an active drug concentration of 10% (w/v) was determined and quantified.

A group of 6 rats was inoculated with a subcutaneous dose of said composition at a ratio of 0,05 ml/Kg of rat.

No edema was observed in any rat.

## Claims

1. An injectable parasiticide composition of a compound having the formula (I): 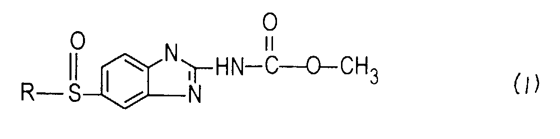 wherein R is a propyl or phenyl group, characterized in that it comprises said compound of formula (I), in a concentration of from to 9 to 11% (w/v) in relation to the final volume, a first solvent selected from ethanol and isopropanol, an inorganic acid and the required amount of propylene glycol up to the 100% of the final volume.

2. The composition as claimed according to claim 1, characterized in that it comprises a compound of formula (I) , in a concentration of 10% (w/v) in relation to the final volume (w/v), a first solvent selected from ethanol and isopropanol, hydrochloric acid; and the required amount of propylene glycol until the 100% final volume is completed.

3. An injectable parasiticide composition of the methyl ester of [(5-propyl-sulphinyl)-1H-benzimidazol-2-il] carbamic acid, named ricobendazole, according to claim 1, characterized in that it comprises ricobendazole in enough amount to achieve a final concentration of about 10% (w/v), a first solvent selected from ethanol and isopropanol, hydrochloric acid, and the required amount of propylene glycol until the 100% final volume is completed.

4. An injectable parasiticide composition of the methyl ester of [(5-phenyl-sulphinyl)-1H-benzimidazol-2-il] carbamic acid, named oxphendazole, according to claim 1, characterized in that it comprises oxphendazole in enough amount to achieve a final concentration of about 10% (w/v), a first solvent selected from ethanol and isopropanol, hydrochloric acid, and the required amount of propylene glycol up until the 100% final volume is completed.

5. The composition according to any of claims 1 to 4, wherein the hydrochloric acid is in a concentration from 3 to 5 % (v/v).

6. The composition according to any of claims 1 to 5, wherein said first solvent is ethanol in enough amount to achieve a final concentration of about 20% (v/v).

7. The composition according to any of claims 1 to 5, wherein said first solvent is isopropanol in enough amount to achieve a final concentration of about 20% (v/v).

8. A process for the preparation of an injectable parasiticide composition of a compound having the formula (I): 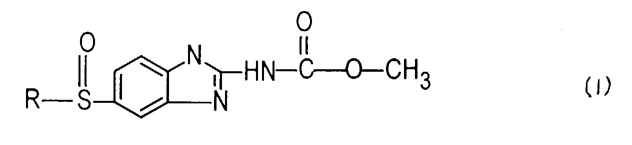 wherein R is a propyl or phenyl group according to claim 1, characterized in that the process comprises adding the enough amount of said compound of formula (I) until achieving a final concentration from 9 to 11% (w/v) in relation to the final volume, to a first solvent selected from ethanol and isopropanol, followed by addition of an inorganic acid; heating at a temperature ranging from 45° to 70°C until total dissolution; adding the required amount of propylene glycol up to the 100% of the final volume.

9. The process according to claim 8, characterized in that a compound of formula (I) is added in enough amount until achieving a final concentration of 10% (w/v) to a first solvent selected from ethanol and isopropanol, followed by addition of hydrochloric acid; heating at a temperature ranging from 45° to 70°C until total dissolution, adding the required amount of propylene glycol up to the 100% of the final volume.

10. The process for the preparation of an injectable parasiticide composition of the methyl ester of [(5-propyl-sulphinyl)- 1H-benzimidazol-2-il] carbamic acid, named ricobendazole according to claim 8, characterized in that the ricobendazole is added in enough amount until achieving a final concentration of about 10% (w/v) to a first solvent selected from ethanol and isopropanol, followed by addition of hydrochloric acid; heating at a temperature ranging from 45° to 70°C until total dissolution, adding the required amount of propylene glycol up to the 100% of the final volume.

11. The process for the preparation of an injectable parasiticide composition of the methyl ester of [(5-phenyl-sulphinyl) 1H-benzimidazol-2-il] carbamic acid, named oxphendazole, according to claim 8, characterized in that the oxphendazole is added in enough amount until achieving a final concentration of about 10% (w/v) to a first solvent selected from ethanol and isopropanol, followed by addition of hydrochloric acid; heating at a temperature ranging from 45° to 70°C until total dissolution, adding the required amount of propylene glycol up to the 100% of the final volume.

12. The process for the preparation of an injectable parasiticide composition of the methyl ester of [(5-propyl-sulphinyl)- 1H-benzimidazol-2-il] carbamic acid, named ricobendazole according to claim 10, characterized in that the ricobendazole is added in enough amount until achieving a final concentration of 10% (w/v) to a first solvent selected from ethanol and isopropanol, followed by addition of hydrochloric acid; heating at a temperature ranging from 45° to 70°C until total dissolution, adding the required amount of propylene glycol up to the 100% of the final volume.

13. The process for the preparation of an injectable parasiticide composition of the methyl ester of [(5-phenyl-sulphinyl)- 1H-benzimidazol-2-il] carbamic acid, named oxphendazole, according to claim 11, characterized in that the oxphendazole is added in enough amount until achieving a final concentration of 10% (w/v) to a first solvent selected from ethanol and isopropanol, followed by addition of hydrochloric acid; heating at a temperature ranging from 45° to 70°C until total dissolution, adding the required amount of propylene glycol up to the 100% of the final volume.

14. The process according to any of claims 8 to 13, wherein the hydrochloric acid is added in enough amount until achieving a final concentration from 3 to 5 % (v/v).

15. The process according to claim 14, wherein the hydrochloric acid is added in enough amount until achieving a final concentration from 3 to 4 % (v/v).

16. The process according to any of claims 8 to 15, wherein said first solvent is ethanol which is in enough amount to achieve a final concentration of about 20% (v/v).

17. The process according to any of claims 8 to 15, wherein said first solvent is isopropanol which is in enough amount to achieve a final concentration of about 20% (v/v).
